(19) Europäisches Patentamt
European Patent Office
Office européen des brevets

(11) **EP 0 818 682 B1**

(12) **EUROPÄISCHE PATENTSCHRIFT**

(45) Veröffentlichungstag und Bekanntmachung des Hinweises auf die Patenterteilung:
**17.10.2001 Patentblatt 2001/42**

(51) Int Cl.$^7$: **G01N 33/72**

(21) Anmeldenummer: **97890130.4**

(22) Anmeldetag: **09.07.1997**

(54) **Verfahren und Messanordnung zur optischen Bestimmung der totalen Hämoglobinkonzentration**

Method and device for the optical measurement of the total hemoglobin concentration

Procédé et dispositif pour la détermination optique de la concentration de l'hémoglobine totale

(84) Benannte Vertragsstaaten:
**DE FR GB**

(30) Priorität: **12.07.1996 AT 126296**

(43) Veröffentlichungstag der Anmeldung:
**14.01.1998 Patentblatt 1998/03**

(60) Teilanmeldung:
**00107256.0 / 1 028 318**

(73) Patentinhaber: **F.HOFFMANN-LA ROCHE AG**
**4070 Basel (CH)**

(72) Erfinder: **Ziegler, Werner, Dipl.-Ing.**
**8043 Graz (AT)**

(74) Vertreter: **Babeluk, Michael, Dipl.-Ing. Mag.**
**Patentanwalt**
**Mariahilfer Gürtel 39/17**
**1150 Wien (AT)**

(56) Entgegenhaltungen:
EP-A- 0 679 890          US-A- 4 997 769
US-A- 5 061 632          US-A- 5 421 329
US-A- 5 692 503

• S. TAKATANI ET AL.: "A miniature hybrid reflection type optical sensor for measurement of hemoglobin content and oxygen saturation of whole blood." IEEE TRANSACTIONS ON BIOMEDICAL ENGINEERING., Bd. 35, Nr. 3, März 1988, Seiten 187-198, XP002082099 NEW YORK US

## Beschreibung

Die Erfindung betrifft ein Verfahren zur optischen Bestimmung der totalen Hämoglobinkonzentration sowie der Sauerstoffsättigung in nicht hämolysiertem Vollblut, unter Verwendung zumindest zweier Messwellenlängen.

Es werden folgende Abkürzungen verwendet:

| | |
|---|---|
| Hb | Hämoglobin |
| tHb | totale Hämoglobinkonzentration (Summe aller aktiven und inaktiven Hämoglobinderivate im Blut) |
| $O_{2sat}$ | Sauerstoffsättigung in Prozent |
| $O_2Hb$ | Oxygeniertes Hb |
| RHb | Deoxygeniertes Hb |
| COHb | Carboxy Hb |
| MetHb | Methämoglobin |
| SulfHb | Sulfhämoglobin |
| $\sigma O$ | Absorptionskoeffizient von $O_2Hb$ |
| $\sigma R$ | Absorptionskoeffizient von RHb |
| $\sigma C$ | Absorptionskoeffizient von COHb |
| $\sigma M$ | Absorptionskoeffizient von MetHb |
| $C_O$ | Konzentration von $O_2Hb$ |
| $C_R$ | Konzentration von RHb |
| $C_C$ | Konzentration von COHb |

Ein Verfahren der eingangs genannten Art ist beispielsweise aus der US-A 5 061 632 bekannt geworden. Das hier beschriebene Oximeter zur Bestimmung von tHb und $O_{2sat}$ weist eine Kapillare zur Aufnahme einer nicht hämolysierten Vollblutprobe auf, welche in die zentrale Bohrung einer zylindrischen Auswerteinheit eingeführt werden kann. Die Auswerteinheit weist in axialem Abstand zwei auf die Zentralbohrung im rechten Winkel angeordnete Radialbohrungen auf, über welche die Messstrahlung aus zwei LEDs zugeführt wird. In axialer Erstreckung zwischen den beiden Radialbohrungen für die Lichtzufuhr ist eine weitere senkrecht auf die Zentralbohrung stehende Radialbohrung angeordnet, welche das von der Probe emittierte Licht einem am Mantel der zylindrischen Auswerteinheit angeordneten Detektor zuführt. Die erste Diode gibt Infrarotstrahlung mit einer Wellenlänge von ca. 800 nm ab und dient zur Bestimmung des tHb. Damit die Messung weitgehend unabhängig von der Verteilung von $O_2Hb$ zu RHb ist, wird die Messung möglichst nahe beim isosbestischen Punkt der Absorptionskoeffizienten von $O_2Hb$ und RHb durchgeführt. Ein derartiger Punkt liegt bei ca. 805 nm (siehe z. B. IEEE Transactions on Biomedical Engineering Vol.35. No. 3, März 1988). Die zweite Diode emittiert rotes Licht einer Wellenlänge von 660 nm und dient zur Bestimmung der Sauerstoffsättigung $O_{2sat}$, da bei dieser Wellenlänge ein signifikanter Unterschied der Absorptionskoeffizienten für $O_2Hb$ und RHb besteht. Die Beiträge der übrigen Hämoglobinderivate (COHb, MetHb und SulfHb) werden beim genannten Verfahren nicht berücksichtigt. Der Fehler für die $O_{2sat}$ Bestimmung liegt dadurch bei ca. 1 bis 15%.

Ein Nachteil beim Verfahren gemäß US-A 5 061 632 besteht darin, dass genau beim isosbestischen Punkt bei $\lambda_i$ = 805 nm keine Standardlichtquellen zur Verfügung stehen und bereits geringe Abweichungen vom isosbestischen Punkt - bedingt durch die gegenläufigen Kurven von $\sigma O$ und $\sigma R$ in diesem Bereich - Fehler bei der Bestimmung des tHb und des $O_{2sat}$ auftreten können.

Ein aus US-A-5 421 329 bekannter Sensor zur Bestimmung der Sauerstoffsättigung verwendet zwei Wellenlängen, von denen eine unterhalb und die andere oberhalb des isosbestischen Punktes liegt. Es wird jedoch kein Verfahren zur Bestimmung der totalen Hämoglobinkonzentration offenbart.

Andere bekannte Verfahren zur Bestimmung der totalen Hämoglobinkonzentration lassen sich wie folgt zusammenfassen:

1) Absorptionsmessungen nach dem Lambert-Beer'schen Gesetz:

Es werden bei einer definierten Anzahl von Messwellenlängen Absorptionsmessungen durchgeführt. Danach wird ein lineares Gleichungssystem ausgewertet, wobei bei Verwendung mehrerer Wellenlänge durch Überbestimmung des Gleichungssystems die Messgenauigkeit erhöht werden kann. Eine Voraussetzung für die Anwendung des Lambert-Beer'schen Gesetzes ist jedoch ein homogenes Medium, d. h. es kann nur hämolysiertes Blut verwendet werden, andernfalls ist das Lambert-Beer'sche Gesetz nur näherungsweise anzuwenden.

2) Aus der WO 94/08237 ist ein Verfahren und eine Vorrichtung für direkte spektrofotometrische Messungen in unverdünntem Vollblut beschrieben, bei welchen Messwellenlängen $\lambda_1$ bis $\lambda_n$ in die Probe eingestrahlt und unter einem großen Detektionswinkel erfasst werden. Es wird somit eine Absorptionsmessung unter Berücksichtigung des gestreuten Anteils des eingestrahlten Lichtes bei einer definierten Anzahl von Messwellenlängen durchgeführt. Nachteiligerweise muss hier ein nichtlineares Gleichungssystem gelöst werden, wobei der Anteil der Nichtlinearität von der Probenstärke abhängig ist.

Aus der DD 203 632 A ist ein Schnellverfahren und eine Einrichtung zur fotometrischen Bestimmung der Konzentration von COHb bekannt geworden. Es wird mit zwei Messwellenlängen operiert, wobei als Lichtquellen Luminszenzdioden mit einer maximalen spektralen Emission bei 565 nm und 940 nm verwendet werden. Als Lichtempfänger dienen Festkörperfotodetektoren, welche unmittelbar auf die Probe gerichtet sind, die als mit dem Untersuchungsmaterial getränktes spezielles Filterpapier vorliegt. Zur Bestimmung der spektralen Absorption werden die Remission und/oder die Transmission ausgewertet, wobei die COHb-Konzentration über einen Rechner ausgegeben wird.

Aus der AT-E 56 271 B ist ein Verfahren zur spektrofotometrischen Bestimmung der Konzentration einer Anzahl von Hämoglobinderivaten in Vollblut bekannt, wobei mehrere unterschiedliche Wellenlängen in die Blutprobe eingestrahlt und Absorptionswerte gemessen werden. Die Konzentrationen der einzelnen Hämoglobinderivate werden durch Lösen eines Gleichungssystems bestimmt.

Weitere fotometrische Methoden zur Messung von Blutbestandteilen sind aus der US-A 5 127 406 und der US-A 5 064 282 bekannt.

Aufgabe der Erfindung ist es, ein Verfahren zur optischen Bestimmung der totalen Hämoglobinkonzentration in nichthämolysiertem Vollblut vorzuschlagen, wobei bei einfachem optischen Aufbau unter möglichst geringem Rechenaufwand brauchbare Messergebnisse erzielt werden sollen. Eine weitere Aufgabe besteht darin, dass das Verfahren auch zur Bestimmung der Sauerstoffsättigung verwendbar sein soll.

Diese Aufgabe wird erfindungsgemäß dadurch gelöst, dass eine erste Messwellenlänge mit $\lambda_1 < 805$ nm sowie eine zweite Messwellenlänge mit $\lambda_2 > 805$ nm verwendet wird, derart, dass für die Absorptionskoeffizienten $\sigma O(\lambda)$ und $\sigma R(\lambda)$ der Hämoglobinderivate $O_2Hb$ und $RHb$ bei den beiden Messwellenlängen $\lambda_1$ und $\lambda_2$ gilt: $\sigma O(\lambda_1)$ ungefähr gleich $\sigma R(\lambda_2)$ und $\sigma R(\lambda_1)$ ungefähr gleich $\sigma O(\lambda_2)$, dass bei den Wellenlängen $\lambda_1$ und $\lambda_2$ die Absorptionswerte $A_1$ und $A_2$ bestimmt werden und die Summe der beiden Absorptionswerte $A_1 + A_2$ eine zur totalen Hämoglobinkonzentration tHb proportionale, von der Sauerstoffsättigung $O_{2sat}$ unabhängige Größe ist. Das erfindungsgemäße Verfahren verwendet zwei isosbestisch symmetrische Wellenlängen, wobei durch die gewählten Wellenlängen mit herkömmlichen Laserdioden die selben Vorteile erzielt werden, welche eine Messung direkt am isosbestischen Punkt ermöglichen würde. Die Absorptionskoeffizienten von $O_2Hb$ und $RHb$ sollten dabei bei den beiden Wellenlängen $\lambda_1$ und $\lambda_2$ jeweils nur um $\pm 5$ % von einander abweichen. Weiters ist die Messung weitgehend unabhängig von der Sauerstoffsättigung der Probe.

Zur Kompensation unterschiedlicher Anregungsintensitäten $I_1$ und $I_2$ bei den Wellenlängen $\lambda_1$ und $\lambda_2$ wird erfindungsgemäß ein Korrekturfaktor f eingeführt, derart, dass die totale Hämoglobinkonzentration tHb der Summe $A_1 + fA_2$ proportional ist, wobei der Faktor f zwischen 0,5 und 2,0 liegt und eine von der gewählten Messanordnung abhängige Kalibriergröße ist.

Vorteilhafte Messwellenlängen für $\lambda_1$ liegen zwischen 780 und 790 nm, vorzugsweise bei $785 \pm 3$ nm sowie für $\lambda_2$ zwischen 830 und 850 nm, vorzugsweise bei $836 \pm 3$ nm. Für die genannten Wellenlängen sind Standardlaserdioden verfügbar (780 bis 785 nm bzw. 840 bis 850 nm) die eine scharf begrenzte Emissionswellenlänge abstrahlen.

Erfindungsgemäße kann die Sauerstoffsättigung $O_{2sat}$ aus den ermittelten Absorptionswerten $A_1$ und $A_2$ näherungsweise durch die Formel $O_{2sat}$ [%] =

$=100.(\sigma R(\lambda_1) .A_2-\sigma R(\lambda_2) .A_1)/((A_1+A_2) . (\sigma R(\lambda_1) - \sigma R(\lambda_2)))$ bestimmt werden. Einer derartigen Bestimmung der Sauerstoffsättigung liegen folgende Überlegungen zugrunde:

$$tHb = C_O+C_R+C_C \quad C_C \ll C_O+C_R \Rightarrow C_C \sim O$$

$$O_{2sat} \, [\%] = 100.C_O/ (C_O+C_R) \sim 100.C_O/tHb$$

$$A_1 = \sigma R_1.C_R + \sigma O_1 C_O + \sigma C_1.C_C \qquad \text{mit } \sigma C_1.C_C \sim 0$$

$$A_2 = \sigma R_2.C_R + \sigma O_2 C_O + \sigma C_2.C_C \qquad \text{mit } \sigma C_2.C_C \sim 0$$

$$\Rightarrow tHb = [A_1(\sigma R_2-\sigma O_2) +A_2(\sigma O_1-\sigma R_1)] / (\sigma R_2.\sigma O_1-\sigma R_1.\sigma O_2)$$

$$\text{mit } \sigma R_1 \sim \sigma O_2, \; \sigma R_2 \sim \sigma O_1$$

$$\Rightarrow tHb = (A_1+A_2)/(\sigma R_2+\sigma R_1 = \underline{K. \, (A_1 \underline{+A_2})}$$

$$C_O = (A_1\sigma R_2-A_2\sigma R_1) /(\sigma R_2.\sigma O_1-\sigma R_1.\sigma O_2)$$

$$O_{2sat} [\%] = 100 \cdot (A_2 \sigma R_1 - A_1 \sigma R_2)/[(A_1+A_2)(\sigma R_1 - \sigma R_2)]$$

Für die Bestimmung der Sauerstoffsättigung kann jedoch auch eine dritte Messwellenlänge verwendet werden, wobei jede Wellenlänge zulässig ist, bei welcher die Absorptionskoeffizienten $\sigma R$ und $\sigma O$ für RHb und $O_2$Hb möglichst große Unterschiede aufweisen. Eine günstige Wellenlänge liegt beispielsweise bei 680 nm (geringer Einfluss von MetHb).

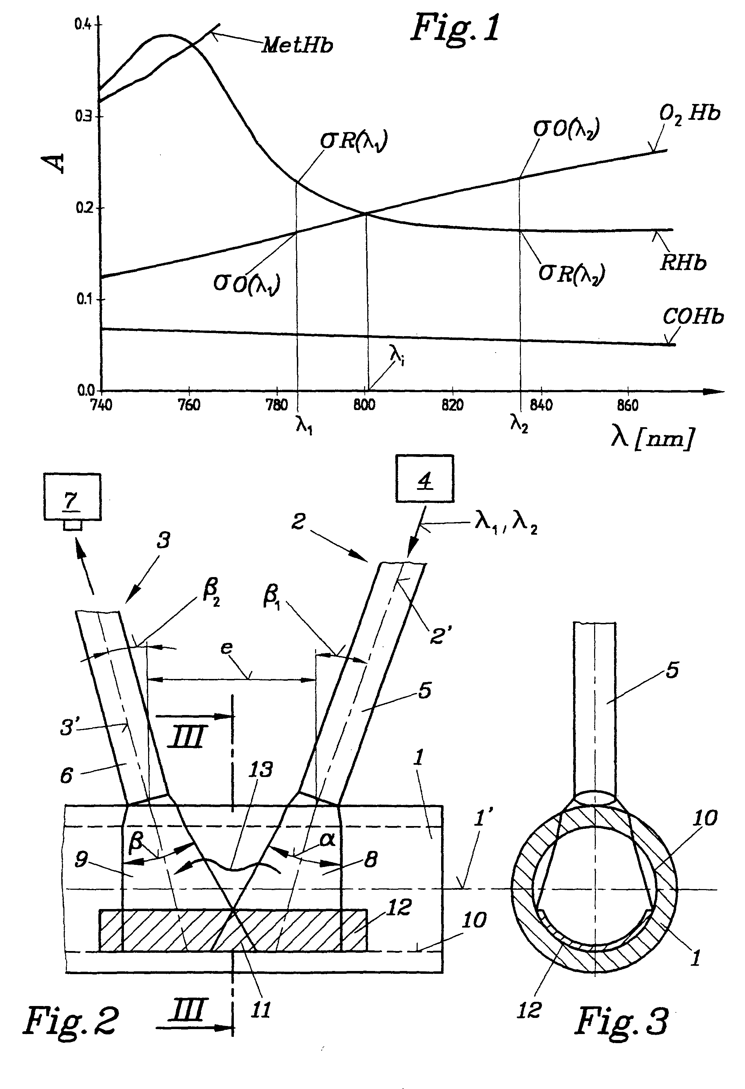

Die Erfindung wird im folgenden anhand von zum Teil schematischen Zeichnungen näher erläutert. Es zeigen Fig. 1 ein Diagramm mit der von der Wellenlänge abhängigen unterschiedlichen Absorption der einzelnen Hämoglobinderivate, Fig. 2 eine Anordnung zur Durchführung des Messverfahrens, Fig. 3 die Messanordnung gemäß Fig. 2, geschnitten entlang der Linie III-III in Fig. 2, sowie Fig. 4 und 5 Diagramme, welche ein von $O_{2sat}$ unabhängiges Summensignal für tHb zeigen.

Aus Fig. 1 sind die unterschiedlichen Beiträge der einzelnen Hämoglobinderivate RHb, $O_2$Hb, COHb und MetHb im Wellenlängenbereich zwischen 740 und 880 nm dargestellt. Ein isosbestischer Punkt für die Hauptbestandteile RHb und $O_2$Hb liegt bei $\lambda_i$ = 805 nm. Aus der Abbildung ist weiters ersichtlich, dass für die eingezeichneten Wellenlängen $\lambda_1$ = 785 nm und $\lambda_2$ = 836 nm $\sigma R(\lambda_1) = \sigma O(\lambda_2)$ und $\sigma O(\lambda_1) = \sigma R(\lambda_2)$ ist. Unter Ausnützung der genannten Bedingung ist die Summe der beiden Absorptionswerte $A_1$ und $A_2$ bei den Wellenlängen $\lambda_1$ und $\lambda_2$ dem tHb der Blutprobe proportional und unabhängig von der Sauerstoffsättigung. Aus Fig. 1 ist weiters ersichtlich, dass der Absorptionskoeffizient von COHb bei beiden Wellenlängen ungefähr gleich groß ist, d. h. $\sigma C(\lambda_1)$ entspricht $\sigma C(\lambda_2)$. Aufgrund des kleinen Wertes des Absorptionskoeffizienten für COHb und der geringen Konzentration von COHb kann deren Produkt für die Berechnung des tHb in erster Näherung vernachlässigt werden.

Die Fig. 2 und 3 zeigen beispielhaft eine Messanordnung zur optischen Bestimmung des tHb in nicht hämolysiertem Vollblut. Die Messanordnung weist einen Kapillarkanal 1 zur Aufnahme einer Blutprobe, eine Anregungseinrichtung 2 zur Einstrahlung von zumindest zwei Messwellenlängen $\lambda_1$ und $\lambda_2$ sowie eine Detektionseinrichtung 3 auf, wobei die optischen Achsen 2' und 3' der Anregungseinrichtung 2 und der Detektionseinrichtung 3 auf den Kapillarkanal 1 gerichtet sind. Die Anregungseinrichtung 2 weist eine Einheit 4, welche die zwei Messwellenlängen $\lambda_1$ und $\lambda_2$ zur Verfügung stellt und Lichtleitmittel, z. B. eine Faseroptik 5, zur Lichtzufuhr auf. Desgleichen besteht die Detektionseinrichtung 3 aus einer Faseroptik 6 und einem Detektor 7. Der Anregungseinrichtung 2 ist bedingt durch die numerische Apertur der Faseroptik 5 ein Strahlöffnungswinkel $\alpha$ für den Anregungslichtkegel 8 zugeordnet. Desgleichen weist die Detektionseinrichtung 3 einen durch einen Akzeptanzwinkel $\beta$ definierten Detektionskegel 9 auf. Beide Kegel 8 und 9 überlappen sich an der Innenwand 10 des Kapillarkanals 1, wenn dieser mit wässriger Lösung gefüllt ist. Um einen genügend großen Überlappungsbereich 11 der beiden Kegel 8 und 9 zu erreichen, kann zumindest eine der Achsen 2' und/oder 3' zur Normale auf die Achse 1' des Kapillarkanals 1 um den Winkel $\beta_1$ und/oder $\beta_2$ geneigt sein. Durch Variation der Entfernung e zwischen der Anregungseinrichtung 2 und der Detektionseinrichtung 3 sowie durch Veränderung der beiden Winkel $\beta_1$ und $\beta_2$ kann die Sensitivität bei der Messung und der Kalibrierung optimiert werden.

Für die Kalibrierung der Messanordnung ist die Innenwand 10 des Kapillarkanals 1 zumindest im Überlappungsbereich 11 der beiden Kegel 8 und 9 mit einer roten Kalibrierfläche 12 versehen. Diese Kalibrierfläche 12 erstreckt sich über ca. 20 bis 40 % des Umfanges der inneren Mantelfläche. Der Innendurchmesser der Kapillare 1 beträgt typischerweise 1 mm.

Nach Abschluss der Kalibrierung wird die Kapillare mit Vollblut gefüllt, wodurch sich die mittlere Eindringtiefe der Messstrahlung auf die obere Hälfte bzw. obere Drittel der Kapillare beschränkt. Die beiden Kegel 8 und 9 überlappen sich dadurch nicht mehr, sodass die Messung durch Streuung an den Blutzellen entlang des Pfeiles 13 erfolgt.

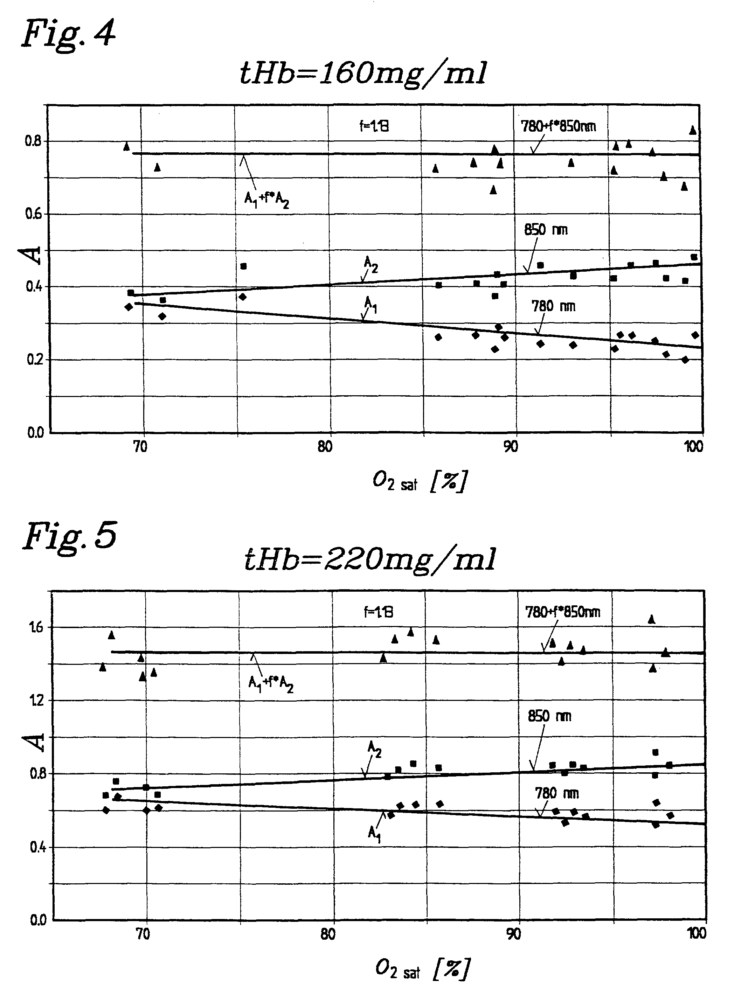

Die Diagramme in den Fig. 4 und 5, bei welchen auf der Ordinate die Absorption A und auf der Abszisse die Sauerstoffsättigung $O_{2sat}$ in Prozent aufgetragen ist, zeigen, dass das Summensignal $A_1+f \cdot A_2$ der Absorptionswerte $A_1$ und $A_2$ bei den Wellenlängen $\lambda_1$= 780 nm und $\lambda_2$= 850 nm für unterschiedliche Werte von $O_{2sat}$ konstant ist. In Fig. 4 beträgt tHb = 160mg/ml in Fig. 5 tHb = 220mg/ml. Der Faktor f = 1.13.

**Patentansprüche**

1. Verfahren zur optischen Bestimmung der totalen Hämoglobinkonzentration (tHb) in nicht hämolysiertem Vollblut, unter Verwendung zumindest zweier Messwellenlängen, **dadurch gekennzeichnet, dass**

    eine erste Messwellenlänge mit $\lambda_1$ < 805 nm sowie eine zweite Messwellenlänge mit $\lambda_2$ > 805 nm verwendet wird, wobei die erste und zweite Meßwellenlänge für die Absorptionskoeffizienten $\sigma O(\lambda)$ und $\sigma R(\lambda)$ der Hämoglobinderivate $O_2$Hb und RHb derart gewählt werden, dass sowohl $\sigma O(\lambda_1)$ ungefähr gleich $\sigma R(\lambda_2)$ ist als auch $\sigma R(\lambda_1)$ ungefähr gleich $\sigma O(\lambda_2)$, ist,
    und dass bei den Wellenlängen $\lambda_1$ und $\lambda_2$ die Absorptionswerte $A_1$ und $A_2$ bestimmt werden und die Summe

der beiden Absorptionswerte $A_1 + A_2$ gebildet wird, um eine zur totalen Hämoglobinkonzentration tHb proportionale und von der Sauerstoffsättigung $O_{2sat}$ unabhängige Größe zu erhalten.

2. Verfahren nach Anspruch 1, **dadurch gekennzeichnet, dass** zur Kompensation unterschiedlicher Anregungsintensitäten $I_1$ und $I_2$ bei den Wellenlängen $\lambda_1$ und $\lambda_2$ ein Korrekturfaktor f eingeführt wird, derart, dass die totale Hämoglobinkonzentration tHb der Summe $A_1 + fA_2$ proportional ist, wobei der Faktor f zwischen 0,5 und 2,0 liegt und eine von der gewählten Messanordnung abhängige Kalibriergröße ist.

3. Verfahren nach Anspruch 1 oder 2, **dadurch gekennzeichnet, dass** $\lambda_1$ zwischen 780 und 790 nm, vorzugsweise bei $785 \pm 3$ nm liegt, sowie dass $\lambda_2$ zwischen 830 und 850 nm, vorzugsweise bei $836 \pm 3$ nm, liegt.

4. Verfahren nach einem der Ansprüche 1 bis 3, **dadurch gekennzeichnet, dass** die Sauerstoffsättigung $O_{2sat}$ aus den ermittelten Absorptionswerten $A_1$ und $A_2$ näherungsweise durch die Formel $O_{2sat}[\%] = 100 \cdot (\sigma R(\lambda_1) \cdot A_2 - \sigma R(\lambda_2) \cdot A_1)/((A_1+A_2) \cdot (\sigma R(\lambda_1) - \sigma R(\lambda_2)))$ bestimmt wird.


**Claims**

1. Method of optically determining the total haemoglobin concentration (tHb) in non-haemolyzed whole blood, using at least two measurement wavelengths, **<u>characterised in that</u>** a first measurement wavelength of $\lambda_1 < 805$ nm and a second measurement wavelength of $\lambda_2 > 805$ nm are employed, said first and second measurement wavelengths being chosen for absorption coefficients $\sigma O(\lambda)$ and $\sigma R(\lambda)$ of the haemoglobin derivatives $O_2Hb$ and RHb such that $\sigma O(\lambda_1)$ will approximately equal $\sigma R(\lambda_2)$ and $\sigma R(\lambda_1)$ will approximately equal $\sigma O(\lambda_2)$, and that the absorption values $A_1$ and $A_2$ are measured at the wavelengths $\lambda_1$ and $\lambda_2$ and the sum of the two absorption values $A_1 + A_2$ is formed to obtain a quantity that is proportional to the total haemoglobin concentration tHb and independent of the oxygen saturation $O_{2sat}$.

2. Method according to claim 1, **characterised in that** for compensation of different excitation intensities $I_1$ and $I_2$ at wavelengths $\lambda_1$ and $\lambda_2$ a correction factor f is introduced, such that the total haemoglobin concentration tHb is proportional to the sum $A_1 + fA_2$, factor f being between 0.5 and 2.0 and representing a calibrating variable dependent on the chosen measuring configuration.

3. Method according to claim 1 or 2, **characterised in that** $\lambda_1$ is between 780 and 790 nm, preferably at $785 \pm 3$ nm, and $\lambda_2$ is between 830 and 850 nm, preferably at $836 \pm 3$ nm.

4. Method according to any of claims 1 to 3, **characterised in that** the oxygen saturation $O_{2sat}$ is approximately determined from the obtained absorption values $A_1$ and $A_2$ by means of the formula $O_{2sat}[\%] = 100 \cdot (\sigma R(\lambda_1) \cdot A_2 - \sigma R(\lambda_2) \cdot A_1)/((A_1+A_2) \cdot (\sigma R(\lambda 1) - \sigma R(\lambda_2)))$.


**Revendications**

1. Procédé de détermination optique de la concentration totale en hémoglobine (tHb) dans du sang complet non hémolysé, en utilisant au moins deux longueurs d'onde de mesure,
   **caractérisé en ce qu'**

   on utilise une première longueur d'onde $\lambda_1 < 805$ nm et une seconde longueur d'onde de mesure $\lambda_2 > 805$ nm, la première et la seconde longueur d'onde de mesure étant choisies pour les valeurs d'absorption $\sigma O(\lambda)$ et $\sigma R(\lambda)$ des dérivés de l'hémoglobine $O_2Hb$ et RHb de façon qu'à la fois $\sigma O(\lambda_1)$ soit sensiblement égal à $\sigma R(\lambda_2)$ et que $\sigma R(\lambda_1)$ soit à peu près égal à $\sigma O(\lambda_2)$, et
   on détermine les valeurs d'absorption ($A_1$ et $A_2$) pour les longueurs d'onde $\lambda_1$ et $\lambda_2$ et
   on forme la somme des deux valeurs d'absorption $A_1 + A_2$ pour avoir une grandeur proportionnelle à la concentration totale d'hémoglobine tHb et qui soit indépendante de la saturation en oxygène $O_{2sat}$.

2. Procédé selon la revendication 1,
   **caractérisé en ce que**

   pour compenser les intensités d'excitation différentes $I_1$ et $I_2$ pour les longueurs d'onde $\lambda_1$ et $\lambda_2$ on introduit

un coefficient de correction f de façon que la concentration totale en hémoglobine tHb soit proportionnelle à la somme $A_1 + fA_2$,

le coefficient f étant compris entre 0,5 et 2,0 et étant une grandeur de calibrage dépendant du dispositif de mesure choisi.

3. Procédé selon l'une quelconque des revendications 1 ou 2,
**caractérisé en ce que**
$\lambda_1$ est compris entre 780 et 790 nm, de préférence de l'ordre de 785 $\pm$ 3 nm, et $\lambda_2$ est compris entre 830 et 850 nm et de préférence égal à 836 $\pm$ 3 nm.

4. Procédé selon l'une quelconque des revendications 1 à 3,
**caractérisé en ce que**
la saturation en oxygène $O_{2sat}$ se détermine à partir des valeurs d'absorption obtenues $A_1$ et $A_2$ approximativement par la formule

$$O_{2sat} [\%] = 100.(\sigma R(\lambda_1) .A_2 - \sigma R(\lambda_2) .A_1)/[(A_1+A_2) . (\sigma R(\lambda_1) - \sigma R(\lambda_2))].$$

## Fig.1

## Fig.2

## Fig.3

## Fig. 4

### $tHb=160mg/ml$

$O_{2\,sat}\ [\%]$

## Fig. 5

### $tHb=220mg/ml$

$O_{2\,sat}\ [\%]$